# EUROPEAN PATENT APPLICATION

(11) **EP 3 881 840 A1**
(43) Date of publication of application: **22.09.2021**
(21) Application number: 20164345.9
(22) Date of filing: 19.03.2020
(51) Int. Cl.: A61K 31/192, A61K 31/435, A61K 31/44, A61K 31/47, A61K 31/495, A61K 45/06, A61P 27/02

(54) **SORTILIN ANTAGONISTS FOR USE INTHE TREATMENT OF DIABETIC RETINOPATHY**

(71) Applicant: Insusense ApS, 2100 Copenhagen (DK)
(72) Inventor: Kjølby, Mads Fuglsang, 2100 Copenhagen (DK); Nykjær, Anders, 8240 Risskov (DK); Little, Paul, 2100 Copenhagen (DK); Jurgeit, Andreas, 2100 Copenhagen (DK)
(74) Representative: Gill Jennings & Every LLP

(57) **Abstract**

The present invention relates to the use of sortilin antagonists, in particular compounds of Formula (I) and preferably Formula (II), in the treatment or prevention of diabetic retinopathy. Also provided are pharmaceutical compositions comprising the sortilin antagonists herein disclosed and methods for the treatment or prevention of diabetic retinopathy in a subject in need thereof.

## Description

### FIELD OF THE INVENTION

The present invention relates to the use of sortilin antagonists, in particular compounds of Formula (I) and preferably Formula (II), in the treatment or prevention of diabetic retinopathy.

### BACKGROUND

Diabetic retinopathy is a complication of diabetes mellitus (DM) that can cause vision loss and blindness as a result of damage to the retina of the eye. The pathogenesis of diabetic retinopathy is thought to involve capillary occlusion and ischaemia in the retinal periphery and hyperperfusion and hyperpermeability in the macular area.¹ Current treatments of diabetic retinopathy include blocking vascular endothelial growth factor (VEGF) signalling, which is an important mediator for these changes, laser photocoagulation (focal or panretinal) or vitrectomy. However, these treatments only treat symptoms and not the underlying disease.2

Sortilin (encoded by *SORT1*) is a type 1 membrane receptor in the vacuolar protein sorting 10 protein (VPS10P) family of sorting receptors, and is most abundantly expressed in the central nervous system. Sortilin has an amino acid sequence according to SEQ ID NO: 1 and comprises a signal peptide, a propeptide, the Vps10p domain, a 10cc domain (10CCa + 10CCb), a transmembrane domain and a large cytoplasmic tail. The luminal domain of sortilin has 6 potential *N*-linked glycosylation sites, whilst the cytoplasmic tail enables for the recruitment of various adapter proteins.

Sortilin binds to a vast number of ligands and membrane receptors and as a result engages in functions known to be important in cellular signalling and sorting. For example, sortilin is involved in signalling by proneurotrophins: the proforms of nerve growth factor (proNGF), brain derived neurotrophic factor (proBDNF), and neurotrophin-3 (proNT3), respectively. In complex with the protein p75NTR, sortilin has been reported to form the receptor for pro-neurotrophin-mediated apoptotic effects leading to degeneration and cell death in cellular and animal models^{3,4,5}. However, sortilin has not been implicated in disease processes of the retina induced by diabetes.

There is an unmet need for new substances useful in the treatment, e.g. by means of delaying progression of symptoms, and prevention of diabetic retinopathy, which address the underlying causes of the disease and not just the symptoms.

### DESCRIPION OF THE FIGURES

Figure 1 depicts the reduction in the "a wave" component of the fERG response 8 weeks after induction of diabetes compared to the baseline, in a diabetic mouse model study using a polyclonal anti-sortilin antibody.
Figure 2 depicts the reduction in the "b wave" component of the fERG response 8 weeks after induction of diabetes compared to the baseline, in a diabetic mouse model study using a polyclonal anti-sortilin antibody.
Figure 3 depicts the reduction in the "a wave" component of the fERG response 8 weeks after induction of diabetes compared to the baseline, in a diabetic mouse model study using Example 8 of the application.
Figure 4 depicts the reduction in the "b wave" component of the fERG response 8 weeks after induction of diabetes compared to the baseline, in a diabetic mouse model study using Example 8 of the application.

### DISCLOSURE OF THE INVENTION

The present invention is based on the surprising discovery that sortilin is implicated in diabetic retinopathy. Without wishing to be bound by theory, it is thought that the protein sortilin is upregulated in the eye in diabetic patients, in particular in the Muller cells. Sortilin can form a trimeric complex with the low-affinity nerve growth factor receptor (p75NTR) and a pro-neurotrophin, such as proNGF, proBDNF or proNT3, resulting in cell death of retinal and ganglion cells while at the same time preventing the conversion of the proform into its mature and trophic form. The present invention provides substances which interfere with the binding of sortilin to pro-neurotrophins, which: 1) prevents the trimeric apoptotic complex of sortilin, p75NTR and the pro-neurotrophin from forming, and 2) prevents sortilin mediated clearance of the pro-neurotrophins, providing a pool of substrate to be converted into the mature and trophic neurotrophin. Thus, sortilin-proneurotrophin interference can be used in protecting the eye from damage resulting from the aforementioned cell death.

It has surprisingly been found that sortilin antagonists, in particular compounds of Formula (I) and (II), may be useful in the treatment or prevention of diabetic retinopathy.

According to a first aspect of the invention, there is provided a sortilin antagonist for use in the prevention or treatment of diabetic retinopathy.

According to another aspect of the invention, there is provided a use of a sortilin antagonist in the manufacture of a medicament for the prevention or treatment of diabetic retinopathy.

According to another aspect of the invention, there is provided a method of preventing or treating diabetic retinopathy by administering a therapeutically effective amount of a sortilin antagonist to a subject in need thereof.

The invention also provides a pharmaceutical formulation comprising the sortilin antagonist of the invention, in particular ophthalmic pharmaceutical formulations.

As used herein, the term "sortilin" may refer to full length sortilin (also referred to as immature sortilin), comprising a signal peptide, a propeptide, a Vps10p domain, a 10CC domain, a transmembrane domain and a large cytoplasmic tail, having an amino acid sequence according to SEQ ID NO: 1 or SEQ ID NO: 2, or it may refer to mature sortilin, comprising a Vps10p domain, a 10CC domain, a transmembrane domain and a large cytoplasmic tail, having an amino acid sequence according to SEQ ID NO: 3, or a naturally occurring fragment, homologue or variant thereof. The term "sortilin" or "sortilin molecule" (used interchangeably herein) as used herein may also include any protein that is functionally related to sortilin, i.e a sortilin-related molecule, for example, SorCS2. Therefore, for the avoidance of doubt, any reference to the term "sortilin" in a use, method or composition of the invention, may not only refer to sortilin as defined above but may also be taken to optionally alternatively refer to a functionally related protein, such as SorCS2. It is understood that the sortilin is capable of interacting with a pro-neurotrophin molecule to form a sortilin/pro-neurotrophin complex. This sortilin/pro-neurotrophin complex may or may not be capable of interacting with a p75NTR molecule to form a trimeric complex comprising sortilin, pro-neurotrophin and p75NTR. It is understood that this trimeric complex may be responsible for adverse biological responses, such as stimulating apoptosis in retinal and ganglion cells.

As used herein, the term "pro-neurotrophin" refers to the larger precursors of neurotrophins, which undergo proteolytic cleavage to yield the mature form of the neurotrophin. Neurotrophins are a family of proteins that induce the survival, development and function of neurons, and are commonly referred to as growth factors. Pro-neurotrophins are biologically active and have distinct roles compared to their neurotrophin counterparts, such as induction of apoptosis. Examples of pro-neurotrophins include proNGF, proBDNF, proNT3, proNT4, preferably the pro-neurotrophin is proNGF, proBDNF or proNT3, even more preferably the pro-neurotrophin is proNGF.

As used herein, the term "sortilin antagonist" refers to a substance (such as an antibody, protein, or other molecule) that interferes with, blocks, or otherwise attenuates the effect of, a sortilin protein binding to a pro-neurotrophin (e.g proNGF, proNT3, proBDNF) and preventing the formation of the trimeric complex between sortilin, p75NTR and the pro-neurotrophin. The term "sortilin antagonist" also includes a substance or agent that interferes with the formation of a high affinity trimeric complex. In the latter scenario, it is recognised that a trimeric complex may be formed in that sortilin can bind to p75NTR (but not proNGF) and p75NTR can simultaneously bind the NGF domain of proNGF. However, the resulting trimeric complex may be of lower affinity for its receptor and as a result have significantly reduced capacity to stimulate apoptosis via the mechanism describe above. The term "sortilin antagonist" also includes a substance or agent that interferes with, blocks, or otherwise attenuates the effect of, a sortilin protein interacting with p75NTR. This interaction may be completely prevented, in which case the trimeric complex is prevented from forming, or only partially prevented, in which case the trimeric complex may be formed but may have reduced biological potency.

Thus, in an embodiment, the sortilin antagonist for use according to the invention may disrupt interaction between a sortilin molecule and a pro-neurotrophin molecule, or disrupt the interaction between a sortilin molecule and a p75NTR molecule.

Preferably, the sortilin antagonist is a sortilin inhibitor. As used herein, the term "sortilin inhibitor" refers to a substance that binds to a sortilin protein, thereby preventing it from binding to a pro-neurotrophin and preventing the formation of the aforementioned trimeric complex, or resulting in the formation of a trimeric complex that is less active or inactive. The term "sortilin inhibitor" may also refer to a soluble fragment of sortilin, or sortilin-related molecule, which can bind competitively to biologically available pro-neurotrophins but prevent formation of an effective trimeric complex. As a result, the sortilin inhibitor results in an inhibition of receptor activation, which would otherwise occur as a result of ligand binding and/or allows for a shift in unbound/biologically available pro-neurotrophin, which in turn can act through a secondary route/receptor either in its pro- or mature form, specifically, but not limited to Trk family receptors.

Preferably, the sortilin antagonist of the present invention prevents the protein-protein interaction between a sortilin protein and a pro-neurotrophin, further preventing the formation of the apoptotic trimeric complex usually formed between sortilin, pro-neurotrophin and the p75NTR receptor, or resulting in the formation of a low affinity trimeric complex, which is biologically less active or inactive or has minimal activity.

It is highly preferred that the sortilin antagonist is a "small molecule". In which case, the sortilin antagonist has a molecular weight of <2000 Da, preferably the sortilin antagonist has a molecular weight of <1000 Da.

In a preferred aspect of the invention, the sortilin antagonist is a compound of Formula (I): 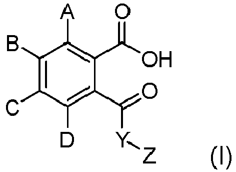 or a pharmaceutically acceptable salt, solvate, hydrate, geometrical isomer, tautomer, optical isomer, N-oxide, and/or prodrug thereof, wherein
Y is selected from the group consisting of -O-, -NR⁵-, and -S-;
Z is selected from the group consisting of optionally substituted C₁-C₆ alkyl, optionally substituted C₆-C₁₀ aryl, and optionally substituted C₁-C₉ heteroaryl;
A, B, C and D are each independently selected from the group consisting of H, optionally substituted C₁-C₆ alkyl, halo, NO₂, optionally substituted C₆-C₁₀ aryl, optionally substituted C₁-C₉ heteroaryl, -OR⁶, NR⁷R⁸, -SR⁹, -C(O)OR¹⁰, -C/O\NR¹¹R¹², -C(O)SR¹³, C(O₂)SR¹⁴;
R¹, R⁴, R⁵, R⁶, R⁹, R¹⁰, R¹³, and R¹⁴ are each independently selected from the group consisting of H and C₁-C₆ alkyl group, wherein the C₁-C₆ alkyl is optionally substituted with one or more halo atoms; and
R², R³, R⁷, R⁸, R¹¹, and R¹² are each independently selected from the group consisting of H and C₁-C₆ alkyl, wherein the C₁-C₆ alkyl is optionally substituted with one or more halo atoms, or R² and R³, R⁷ and R⁸, and/or R¹¹ and R¹² can be taken together with the nitrogen atom to which they are attached to from a 5- or 6-membered heterocycle, optionally substituted with one or more halo atoms;

Compounds of Formula (I) act as sortilin antagonists by binding to sortilin. They are therefore useful in the treatment or prevention of diabetic retinopathy.

It is highly preferred that Y is -NR⁵-, more preferably Y is -NH-. In a particularly preferred aspect of the invention, the compound of Formula (I) is a compound of Formula (II):

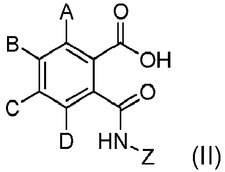

Preferred compounds of Formula (I) and (II), as detailed below, may exhibit increased binding affinity to sortilin and therefore increased efficacy in the treatment or prevention of diabetic retinopathy.

In a preferred aspect of the invention, A and D are H.

In another preferred aspect of the invention, B and C are independently selected from the group consisting of H, halo, CF₃, NO₂, and C₁-C₃ alkyl.

It is more preferred that C is H.

It is also more preferred that B is selected from the group consisting of halo and CF3, more preferably B is Br or CF₃.

In another preferred aspect of the invention, Z is an optionally substituted C₆-C₁₀ aryl or an optionally substituted C₁-C₉ heteroaryl.

It is preferred that the optionally substituted C₆-C₁₀ aryl is an optionally substituted phenyl.

Particularly preferred optionally substituted C₁-C₉ heteroaryl groups are optionally substituted 5- or 6-membered heteroaryl groups in which 1 to 5 of the ring atoms are carbon and one or more of the ring atoms are a heteroatom, more preferably optionally substituted 6-membered heteroaryl groups in which 1 to 5 of the ring atoms are carbon and one or more of the ring atoms are a heteroatom.

It is preferred that the ring atoms of the optionally substituted 6-membered are selected from the group consisting of carbon and nitrogen. In which case, particularly preferred examples of optionally substituted C₁-C₉ heteroaryl groups are optionally substituted pyridyl, pyridazinyl, pyrimidinyl, pyrazinyl, and triazinyl. More preferred optionally substituted C₁-C₉ heteroaryl groups are optionally substituted pyridyl and pyrimidinyl.
Therefore, in a particularly preferred aspect of the invention, Z is an optionally substituted phenyl, an optionally substituted pyridyl, or an optionally substituted pyrimidinyl.

Z may be substituted with one or more substituents independently selected from the group consisting of -OR¹⁵, halo, and C₁-C₄ alkyl, wherein R¹⁵ is H, halo, and C₁-C₃ alkyl. More preferably, Z is substituted with one or more substituents selected from the group consisting of Cl, -OMe, and Me.

In addition to the above-mentioned preferred optional substituents, an alkylene group may be attached to two adjacent atoms of group Z to form a 5- or 6-membered partially saturated or saturated ring, optionally wherein the alkylene group is substituted with one or more halo atoms. Examples of such fused bicyclic ring systems are shown below in the following two examples of group Z: 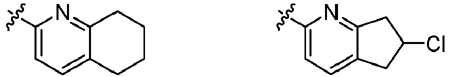 Particular combinations of Z and its substituents may be particularly preferred, as detailed below.

In a particularly preferred aspect of the invention, Z is C₆-C₁₀ aryl or C₁-C₉ heteroaryl. Each of C₆-C₁₀ aryl and C₁-C₉ heteroaryl are independently substituted with one or more substituents independently selected from the group consisting of -OR¹⁵, halo, and C₁-C₄ alkyl, wherein R¹⁵ is H, halo, or C₁-C₃ alkyl, preferably one or more substituents independently selected from the group consisting of Cl, -OMe, and Me; and/or an alkylene group is attached to two adjacent atoms of group Z to form a 5- or 6-membered partially saturated or saturated ring, optionally wherein the alkylene group is substituted with one or more halo atoms.

In an even more preferred aspect of the invention, Z is phenyl, pyridyl, or pyrimidinyl. Each of phenyl, pyridyl, and pyrimidinyl are independently substituted with one or more substituents independently selected from the group consisting of -OR¹⁵, halo, and C₁-C₄ alkyl, wherein R¹⁵ is H, halo, or C₁-C₃ alkyl, preferably one or more substituents independently selected from the group consisting of Cl, -OMe, and Me; and/or an alkylene group is attached to two adjacent atoms of group Z to form a 5- or 6-membered partially saturated or saturated ring, optionally wherein the alkylene group is substituted with one or more halo atoms.

Particular compounds for use according to the invention are:
- 2-[(6-methylpyridin-2-yl)carbamoyl]benzoic acid;
- 2-methyl-6-[(6-methylpyridin-2-yl)carbamoyl]benzoic acid;
- 5-bromo-2-[(6-methylpyridin-2-yl)carbamoyl]benzoic acid;
- 5-chloro-2-[(6-methylpyridin-2-yl)carbamoyl]benzoic acid;
- 5-methyl-2-[(6-methylpyridin-2-yl)carbamoyl]benzoic acid;
- 2-[(6-methylpyridin-2-yl)carbamoyl]-5-(propan-2-yl)benzoic acid;
- 2-[(6-methylpyridin-2-yl)carbamoyl]-5-(trifluoromethyl)benzoic acid;
- 2-[(6-methylpyridin-2-yl)carbamoyl]-5-nitrobenzoic acid;
- 4-[(6-methylpyridin-2-yl)carbamoyl]-[1,1'-biphenyl]-3-carboxylic acid;
- 4-bromo-2-[(6-methylpyridin-2-yl)carbamoyl]benzoic acid;
- 4-chloro-2-[(6-methylpyridin-2-yl)carbamoyl]benzoic acid;
- 4-methyl-2-[(6-methylpyridin-2-yl)carbamoyl]benzoic acid;
- 2-[(6-methylpyridin-2-yl)carbamoyl]-4-(trifluoromethyl)benzoic acid;
- 4,5-dichloro-2-[(6-methylpyridin-2-yl)carbamoyl]benzoic acid;
- 4,5-dimethyl-2-[(6-methylpyridin-2-yl)carbamoyl]benzoic acid;
- 3-methyl-2-[(6-methylpyridin-2-yl)carbamoyl]benzoic acid;
- 5-bromo-2-[(butan-2-yl)carbamoyl]benzoic acid;
- 5-bromo-2-[(propan-2-yl)carbamoyl]benzoic acid;
- 5-bromo-2-(phenylcarbamoyl)benzoic acid;
- 5-bromo-2-[(3-methylphenyl)carbamoyl]benzoic acid;
- 5-bromo-2-[(pyridin-2-yl)carbamoyl]benzoic acid;
- 5-bromo-2-[(6-chloropyridin-2-yl)carbamoyl]benzoic acid;
- 5-bromo-2-[(4-methylpyridin-2-yl)carbamoyl]benzoic acid;
- 5-bromo-2-[(3-methylpyridin-2-yl)carbamoyl]benzoic acid;
- 5-bromo-2-[(6-methylpyridin-3-yl)carbamoyl]benzoic acid;
- 5-bromo-2-[(2-methylpyridin-4-yl)carbamoyl]benzoic acid;
- 5-bromo-2-[(2-methylpyrimidin-4-yl)carbamoyl]benzoic acid;
- 2-[(6-methoxypyridin-2-yl)carbamoyl]-5-(trifluoromethyl)benzoic acid;
- 2-[(5,6-dimethylpyridin-2-yl)carbamoyl]-5-(trifluoromethyl)benzoic acid;
- 2-[(5,6,7,8-tetrahydroquinolin-2-yl)carbamoyl]-5-(trifluoromethyl)benzoic acid,
or a pharmaceutically acceptable salt, solvate, hydrate, tautomer, optical isomer, N-oxide, and/or prodrug thereof.

Particularly preferred compounds for use according to the invention are:
- 5-bromo-2-[(6-methylpyridin-2-yl)carbamoyl]benzoic acid;
- 5-chloro-2-[(6-methylpyridin-2-yl)carbamoyl]benzoic acid;
- 5-methyl-2-[(6-methylpyridin-2-yl)carbamoyl]benzoic acid;
- 2-[(6-methylpyridin-2-yl)carbamoyl]-5-(propan-2-yl)benzoic acid;
- 2-[(6-methylpyridin-2-yl)carbamoyl]-5-(trifluoromethyl)benzoic acid;
- 2-[(6-methylpyridin-2-yl)carbamoyl]-5-nitrobenzoic acid;
- 4-bromo-2-[(6-methylpyridin-2-yl)carbamoyl]benzoic acid;
- 2-[(6-methylpyridin-2-yl)carbamoyl]-4-(trifluoromethyl)benzoic acid;
- 4,5-dichloro-2-[(6-methylpyridin-2-yl)carbamoyl]benzoic acid;
- 4,5-dimethyl-2-[(6-methylpyridin-2-yl)carbamoyl]benzoic acid;
- 5-bromo-2-[(3-methylphenyl)carbamoyl]benzoic acid;
- 5-bromo-2-[(pyridin-2-yl)carbamoyl]benzoic acid;
- 5-bromo-2-[(6-chloropyridin-2-yl)carbamoyl]benzoic acid;
- 5-bromo-2-[(4-methylpyridin-2-yl)carbamoyl]benzoic acid;
- 5-bromo-2-[(2-methylpyridin-4-yl)carbamoyl]benzoic acid;
- 5-bromo-2-[(2-methylpyrimidin-4-yl)carbamoyl]benzoic acid;
- 2-[(6-methoxypyridin-2-yl)carbamoyl]-5-(trifluoromethyl)benzoic acid;
- 2-[(5,6-dimethylpyridin-2-yl)carbamoyl]-5-(trifluoromethyl)benzoic acid;
- 2-[(5,6,7,8-tetrahydroquinolin-2-yl)carbamoyl]-5-(trifluoromethyl)benzoic acid;
or a pharmaceutically acceptable salt, solvate, hydrate, tautomer, optical isomer, N-oxide, and/or prodrug thereof.

According to another aspect of the invention, there is provided a pharmaceutical formulation comprising a sortilin antagonist according to the invention and one or more pharmaceutically acceptable carriers or excipients, for use in the treatment or prevention of diabetic retinopathy.

In a preferred aspect of the invention, the pharmaceutical formulation is an ophthalmic formulation.

The compounds for use according to the invention may include isotopically-labelled and/or isotopically-enriched forms of the compounds. The compounds for use according to the invention herein may contain unnatural proportions of atomic isotopes at one or more of the atoms that constitute such compounds. Examples of isotopes that can be incorporated into the disclosed compounds include isotopes of hydrogen, carbon, nitrogen, oxygen, phosphorus, sulfur, chlorine, such as ²H, ³H, ¹¹C, ¹³C, ¹⁴C, ¹³N, ¹⁵O, ¹⁷O, ³²P, ³⁵S, ¹⁸F, ³⁶Cl.

The compounds for use according to the invention may be used as such or, where appropriate, as pharmacologically acceptable salts (acid or base addition salts) thereof. The pharmacologically acceptable addition salts mentioned below are meant to comprise the therapeutically active non-toxic acid and base addition salt forms that the compounds are able to form. Compounds that have basic properties can be converted to their pharmaceutically acceptable acid addition salts by treating the base form with an appropriate acid. Exemplary acids include inorganic acids, such as hydrogen chloride, hydrogen bromide, hydrogen iodide, sulphuric acid, phosphoric acid; and organic acids such as formic acid, acetic acid, propanoic acid, hydroxyacetic acid, lactic acid, pyruvic acid, glycolic acid, maleic acid, malonic acid, oxalic acid, benzenesulphonic acid, toluenesulphonic acid, methanesulphonic acid, trifluoroacetic acid, fumaric acid, succinic acid, malic acid, tartaric acid, citric acid, salicylic acid, p-aminosalicylic acid, pamoic acid, benzoic acid, ascorbic acid and the like. Exemplary base addition salt forms are the sodium, potassium, calcium salts, and salts with pharmaceutically acceptable amines such as, for example, ammonia, alkylamines, benzathine, and amino acids, such as, e.g. arginine and lysine. The term addition salt as used herein also comprises solvates which the compounds and salts thereof are able to form, such as, for example, hydrates, alcoholates and the like.

Throughout the present disclosure, a given chemical formula or name shall also encompass all pharmaceutically acceptable salts, solvates, hydrates, geometrical isomers, tautomers, optical isomers, N-oxides, and/or prodrug forms thereof. It is to be understood that the compounds for use according to the invention include any and all hydrates and/or solvates of the compound formulas. It is appreciated that certain functional groups, such as the hydroxy, amino, and like groups form complexes and/or coordination compounds with water and/or various solvents, in the various physical forms of the compounds. Accordingly, the above formulas are to be understood to include and represent those various hydrates and/or solvates. Compounds for use according to the invention also include tautomeric forms. Tautomeric forms result from the swapping of a single bond with an adjacent double bond together with the concomitant migration of a proton. Tautomeric forms include prototropic tautomers which are isomeric protonation states having the same empirical formula and total charge. Example prototropic tautomers include ketone - enol pairs, amide - imidic acid pairs, lactam - lactim pairs, amide - imidic acid pairs, enamine - imine pairs, and annular forms where a proton can occupy two or more positions of a heterocyclic system, for example, 1H- and 3H-imidazole, 1H, 2H- and 4H- 1,2,4-triazole, 1H- and 2H- isoindole, and 1H- and 2H-pyrazole. Tautomeric forms can be in equilibrium or sterically locked into one form by appropriate substitution.

The compounds described herein can be asymmetric (e.g. having one or more stereocenters). All stereoisomers, such as enantiomers and diastereomers, are intended unless otherwise indicated. Compounds for use according to the present invention that contain asymmetrically substituted carbon atoms can be isolated in optically active or racemic forms. Methods on how to prepare optically active forms from optically active starting materials are known in the art, such as by resolution of racemic mixtures or by stereoselective synthesis. Many geometric isomers of olefins, C=N double bonds, and the like can also be present in the compounds described herein, and all such stable isomers are contemplated in the present invention. Cis- and trans-geometric isomers of the compounds for use according to the present invention are described and may be isolated as a mixture of isomers or as separated isomeric forms.

In the case of the compounds, which contain an asymmetric carbon atom, the invention relates to the D form, the L form, and D, L mixtures and also, where more than one asymmetric carbon atom is present, to the diastereomeric forms. Those compounds for use according to the invention which contain asymmetric carbon atoms, and which as a rule accrue as racemates, can be separated into the optically active isomers in a known manner, for example using an optically active acid. However, it is also possible to use an optically active starting substance from the outset, with a corresponding optically active or diastereomeric compound then being obtained as the end product.

The term "prodrugs" refers to compounds that may be converted under physiological conditions or by solvolysis to a biologically active compound for use according to the invention. A prodrug may be inactive when administered to a subject in need thereof, but is converted in vivo to an active compound of the invention. Prodrugs are typically rapidly transformed in vivo to yield the parent compound of the invention, e.g. by hydrolysis in the blood. The prodrug compound usually offers advantages of solubility, tissue compatibility or delayed release in a mammalian organism (see Silverman, R. B., The Organic Chemistry of Drug Design and Drug Action, 2nd Ed., Elsevier Academic Press (2004), page 498 to 549). Prodrugs of a compound for use according to the invention may be prepared by modifying functional groups, such as a hydroxy, amino or mercapto groups, present in a compound for use according to the invention in such a way that the modifications are cleaved, either in routine manipulation or in vivo, to the parent compound for use according to the invention. Examples of prodrugs include, but are not limited to, acetate, formate and succinate derivatives of hydroxy functional groups or phenyl carbamate derivatives of amino functional groups.

The term "treatment" as used herein may include prophylaxis of the named disorder or condition, or amelioration or elimination of the disorder once it has been established. The term "prevention" refers to prophylaxis of the named disorder or condition.

Methods delineated herein include those wherein the subject is identified as in need of a particular stated treatment. Identifying a subject in need of such treatment can be in the judgment of a subject or a health care professional and can be subjective (e.g. opinion) or objective (e.g. measurable by a test or diagnostic method).

In other aspects, the methods herein include those further comprising monitoring subject response to the treatment administrations. Such monitoring may include periodic imaging or sampling of subject tissue, fluids, specimens, cells, proteins, chemical markers, genetic materials, etc. as markers or indicators of the treatment regimen. In other methods, the subject is pre-screened or identified as in need of such treatment by assessment for a relevant marker or indicator of suitability for such treatment.

The invention provides a method of monitoring treatment progress. The method includes the step of determining a level of diagnostic marker (Marker) (e.g. any target or cell type delineated herein modulated by a compound herein) or diagnostic measurement (e.g., screen, assay) in a subject suffering from or susceptible to a disorder or symptoms thereof delineated herein, in which the subject has been administered a therapeutic amount of a compound herein sufficient to treat the disease or symptoms thereof. The level of Marker determined in the method can be compared to known levels of Marker in either healthy normal controls or in other afflicted patients to establish the subject's disease status. In preferred embodiments, a second level of Marker in the subject is determined at a time point later than the determination of the first level, and the two levels are compared to monitor the course of disease or the efficacy of the therapy. In certain preferred embodiments, a pre-treatment level of Marker in the subject is determined prior to beginning treatment according to this invention; this pre-treatment level of Marker can then be compared to the level of Marker in the subject after the treatment commences, to determine the efficacy of the treatment.

A level of Marker or Marker activity in a subject may be determined at least once. Comparison of Marker levels, e.g., to another measurement of Marker level obtained previously or subsequently from the same patient, another patient, or a normal subject, may be useful in determining whether therapy according to the invention is having the desired effect, and thereby permitting adjustment of dosage levels as appropriate. Determination of Marker levels may be performed using any suitable sampling/expression assay method known in the art or described herein. Preferably, a tissue or fluid sample is first removed from a subject. Examples of suitable samples include blood, urine, tissue, mouth or cheek cells, and hair samples containing roots. Other suitable samples would be known to the person skilled in the art. Determination of protein levels and/or mRNA levels (e.g., Marker levels) in the sample can be performed using any suitable technique known in the art, including, but not limited to, enzyme immunoassay, is ELISA, radiolabeling/assay techniques, blotting/chemiluminescence methods, real-time PCR, and the like.

For clinical use, the compounds disclosed herein are formulated into pharmaceutical compositions (or formulations) for various modes of administration. It will be appreciated that compounds for use according to the invention may be administered together with a physiologically acceptable carrier, excipient, and/or diluent (i.e. one, two, or all three of these). The pharmaceutical compositions disclosed herein may be administered by any suitable route, preferably by oral, ocular (including intravitreal), rectal, nasal, topical (including buccal and sublingual), sublingual, transdermal, intrathecal, transtympanic transmucosal or parenteral (including subcutaneous, intramuscular, intravenous and intradermal) administration. Preferably, the pharmaceutical compositions are administered ocularly. Other formulations may conveniently be presented in unit dosage form, e.g., tablets and sustained release capsules, and in liposomes, and may be prepared by any methods well known in the art of pharmacy. Pharmaceutical formulations are usually prepared by mixing the active substance, or a pharmaceutically acceptable salt thereof, with conventional pharmaceutically acceptable carriers, diluents or excipients. Examples of excipients are water, gelatin, gum arabicum, lactose, microcrystalline cellulose, starch, sodium starch glycolate, calcium hydrogen phosphate, magnesium stearate, talcum, colloidal silicon dioxide, and the like. Such formulations may also contain other pharmacologically active agents, and conventional additives, such as stabilizers, wetting agents, emulsifiers, flavouring agents, buffers, and the like. Usually, the amount of active compounds is between 0.1-95% by weight of the preparation, preferably between 0.2-20% by weight in preparations for parenteral use and more preferably between 1-50% by weight in preparations for oral administration. The formulations can be further prepared by known methods such as granulation, compression, microencapsulation, spray coating, etc. The formulations may be prepared by conventional methods in the dosage form of tablets, eye drops, creams, capsules, granules, powders, syrups, suspensions, suppositories or injections. Liquid formulations may be prepared by dissolving or suspending the active substance in water or other suitable vehicles. Tablets and granules may be coated in a conventional manner. To maintain therapeutically effective plasma concentrations for extended periods of time, compounds disclosed herein may be incorporated into slow release formulations.

The dose level and frequency of dosage of the specific compound will vary depending on a variety of factors including the potency of the specific compound employed, the metabolic stability and length of action of that compound, the patient's age, body weight, general health, sex, diet, mode and time of administration, rate of excretion, drug combination, the severity of the condition to be treated, and the patient undergoing therapy. The daily dosage may, for example, range from about 0.001 mg to about 100 mg per kilo of body weight, administered singly or multiply in doses, e.g. from about 0.01 mg to about 25 mg each. Normally, such a dosage is given orally but parenteral administration may also be chosen.

### DEFINITIONS

"Optional" or "optionally" means that the subsequently described event or circumstance may, but need not, occur, and that the description includes instances where the event or circumstance occurs and instances in which it does not.

The term "C₁-C₆ alkyl" denotes a straight, branched or cyclic or partially cyclic alkyl group having from 1 to 6 carbon atoms, i.e. 1, 2, 3, 4, 5 or 6 carbon atoms. For the "C₁-C₆ alkyl" group to comprise a cyclic portion it should be formed of 3 to 6 carbon atoms. For parts of the range "C₁-C₆ alkyl" all subgroups thereof are contemplated, such as C₁-C₅ alkyl, C₁-C₄ alkyl, C₁-C₃ alkyl, C₁-C₂ alkyl, C₁ alkyl, C₂-C₆ alkyl, C₂-C₅ alkyl, C₂-C₄ alkyl, C₂-C₃ alkyl, C₂ alkyl, C₃-C₆ alkyl, C₃-C₅ alkyl, C₃-C₄ alkyl, C₃ alkyl, C₄-C₆ alkyl, C₄-C₅ alkyl, C₄ alkyl, C₅-C₆ alkyl, C₅ alkyl, and C₆ alkyl. Examples of "C₁-C₆ alkyl" include methyl, ethyl, n-propyl, isopropyl, cyclopropyl, n-butyl, isobutyl, sec-butyl, t-butyl, cyclobutyl, cyclopropylmethyl, and straight, branched or cyclic or partially cyclic pentyl and hexyl etc.

When a term denotes a range, for instance "1 to 6 carbon atoms" in the definition of C₁-C₆ alkyl, each integer is considered to be disclosed, i.e. 1, 2, 3, 4, 5 and 6.

The term "halo atom" means a halogen atom, and is preferably F, Cl, Br or I.

The term "C₆-C₁₀ aryl" denotes an aromatic monocyclic or fused bicyclic hydrocarbon ring system comprising 6 to 10 ring atoms. Examples of "C₆-C₁₀ aryl" groups include phenyl, indenyl, naphthyl, and naphthalene.

The term "C₁-C₉ heteroaryl" denotes an aromatic monocyclic or fused bicyclic heteroaromatic ring system having 5 to 10 ring atoms in which 1 to 9 of the ring atoms are carbon and one or more of the ring atoms are selected from nitrogen, sulphur, and oxygen. Examples of "C₁-C₉ heteroaryl" include furyl, pyrrolyl, thienyl, oxazolyl, isoxazolyl, imidazolyl, thiazolyl, isothiazolyl, pyridinyl, pyrimidinyl, tetrazolyl, quinazolinyl, indolyl, indolinyl, isoindolyl, isoindolinyl, pyrazolyl, pyridazinyl, pyrazinyl, quinolinyl, quinoxalinyl, thiadiazolyl, benzofuranyl, 2,3-dihydrobenzofuranyl, 1,3-benzodioxolyl, 1,4-benzodioxinyl, 2,3-dihydro-1,4-benzodioxinyl, benzothiazolyl, benzimidazolyl, benzothiadiazolyl, benzotriazolyl and chromanyl.

"An effective amount" refers to an amount of a compound for use according to the invention that confers a therapeutic effect on the treated subject. The therapeutic effect may be objective (i.e. measurable by some test or marker) or subjective (i.e. subject gives an indication of or feels an effect).

As used herein, the terms "administration" or "administering" mean a route of administration for a compound disclosed herein. Exemplary routes of administration include, but are not limited to, oral, ocular (such as intravitreal), topical, intravenous, intraperitoneal, intraarterial, and intramuscular. The preferred route of administration can vary depending on various factors, e.g. the components of the pharmaceutical composition comprising a compound disclosed herein, site of the potential or actual disease and severity of disease.

The terms "subject" and "patient" are used herein interchangeably. They refer to a human or another mammal (e.g., mouse, rat, rabbit, dog, cat, cattle, swine, sheep, horse or primate) that can be afflicted with or is susceptible to a disease or disorder but may or may not have the disease or disorder. It is preferred that the subject is human.

Compounds for use according to the invention may be disclosed by the name or chemical structure. If a discrepancy exists between the name of a compound and its associated chemical structure, then the chemical structure prevails.

### PREPARATION OF COMPOUNDS OF THE INVENTION

The compounds of Formulae (I) and (II) disclosed herein may be prepared by, or in analogy with, conventional methods. Appropriate reaction conditions for the individual reaction steps are known to a person skilled in the art. The necessary starting materials for preparing the compounds of Formulae (I) and (II) are either commercially available, or may be prepared by methods known in the art.

The compounds of Formulae (I) and (II) may possess one or more chiral carbon atoms, and they may therefore be obtained in the form of optical isomers, e.g., as a pure enantiomer, or as a mixture of enantiomers (racemate) or as a mixture containing diastereomers. The separation of mixtures of optical isomers to obtain pure enantiomers is well known in the art and may, for example, be achieved by fractional crystallization of salts with optically active (chiral) acids or by chromatographic separation on chiral columns.

Particular experimental procedures for examples of the invention are described below. The processes may be carried out to give a compound of the invention in the form of a free base or as an acid addition salt. A pharmaceutically acceptable acid addition salt may be obtained by dissolving the free base in a suitable organic solvent and treating the solution with an acid, in accordance with conventional procedures for preparing acid addition salts from base compounds. Examples of addition salt forming acids are mentioned above. The chemicals used in the synthetic routes delineated herein may include, for example, solvents, reagents, catalysts, and protecting group and deprotecting group reagents. Examples of protecting groups are t-butoxycarbonyl (Boc), benzyl and trityl(triphenylmethyl). The methods described below may also additionally include steps, either before or after the steps described specifically herein, to add or remove suitable protecting groups in order to ultimately allow synthesis of the compounds. In addition, various synthetic steps may be performed in an alternate sequence or order to give the desired compounds.

Synthetic chemistry transformations and protecting group methodologies (protection and deprotection) useful in synthesizing applicable compounds are known in the art and include, for example, those described in R. Larock, Comprehensive Organic Transformations, VCH Publishers (1989); T.W. Greene and P.G.M. Wuts, Protective Groups in Organic Synthesis, 3rd Ed., John Wiley and Sons (1999); L. Fieser and M. Fieser, Fieser and Fieser's Reagents for Organic Synthesis, John Wiley and Sons (1994); and L. Paquette, ed., Encyclopedia of Reagents for Organic Synthesis, John Wiley and Sons (1995) and subsequent editions thereof.

The invention will now be further illustrated by the following non-limiting examples. The specific examples below are to be construed as merely illustrative, and not limitative of the remainder of the disclosure in any way whatsoever. Without further elaboration, it is believed that one skilled in the art can, based on the description herein, utilize the present invention to its fullest extent. All references and publications cited herein are hereby incorporated by reference in their entirety.

### GENERAL SYNTHETIC PROCEDURE 1

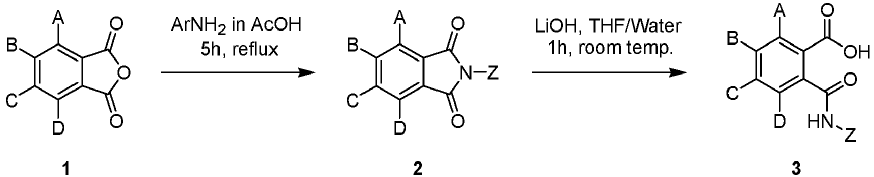

In general synthetic procedure 1, phthalic anhydride **1** undergoes condensation-*cyclo*-dehydartion to produce phthalimide **2** followed by hydrolysis to form phthalamic acid **3.** The regioisomers of phthalamic acid **3** are then separated via preparative HPLC.⁶

### GENEREAL SYNTHETIC PROCEDURE 2

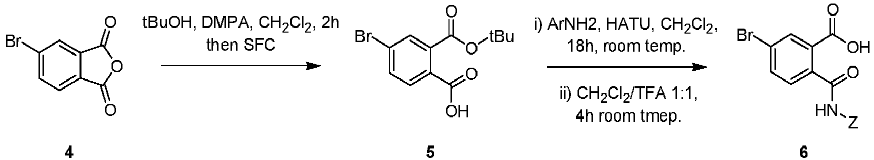

In general, synthetic procedure 2, phthalic anhydride **4** is reacted with *tert*-butanol followed by separation of the regioisomeric mono esters by supercritical fluid chromatography (SFC) to produce phthalate mono-*t*-butyl ester **5.** Phthalate mono-*t*-butyl ester **5** subsequently undergoes coupling deprotection resulting in phthalamic acid **6**.⁶

Palladium-catalysed coupling could be used to replace the bromine of phthalate mono-*t*-butyl ester **5** or phthalamic acid **6** with alternative substituents.⁶

### EXAMPLES

### Examples 1 and 2

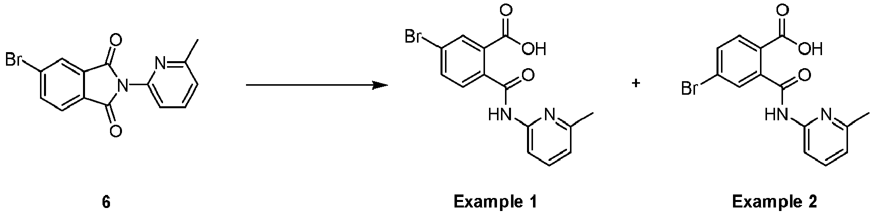

Phthalamide **6** was degraded under assay conditions (see description of Neurotensin (NTS) scintillation proximity assay (SPA) below) to afford the two corresponding phthalimide hydrolysis products, 5-bromo-2-[(6-methylpyridin-2-yl)carbamoyl]benzoic acid (**Example 1**) and 4-bromo-2-[(6-methylpyridin-2-yl)carbamoyl]benzoic acid (**Example 2**).⁶

Alternatively, Examples 1 and 2 may be prepared in accordance with general synthetic procedures 1 and/or 2.

### Examples 3 to 30

Examples 3 to 30 can be prepared in accordance with general synthetic procedures 1 and/or 2.

| **Ex**. | **Compound Name** | **Structure** |
|---|---|---|
| 3 | 2-[(6-methylpyridin-2-yl)carbamoyl]benzoic acid | 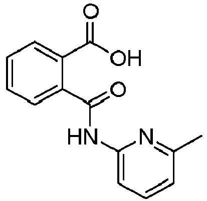 |
| 4 | 2-methyl-6-[(6-methylpyridin-2-yl)carbamoyl]benzoic acid | 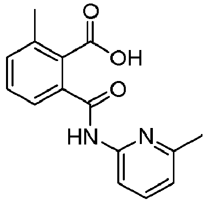 |
| 5 | 5-chloro-2-[(6-methylpyridin-2-yl)carbamoyl]benzoic acid | 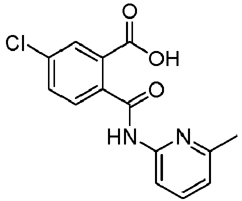 |
| 6 | 5-methyl-2-[(6-methylpyridin-2-yl)carbamoyl]benzoic acid | 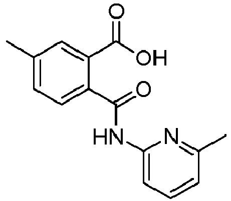 |
| 7 | 2-[(6-methylpyridin-2-yl)carbamoyl]-5-(propan-2-yl)benzoic acid | 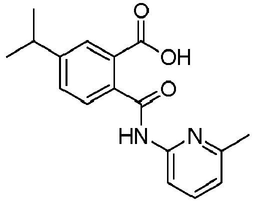 |
| 8 | 2-[(6-methylpyridin-2-yl)carbamoyl]-5-(trifluoromethyl)benzoic acid | 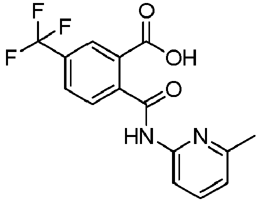 |
| 9 | 2-[(6-methylpyridin-2-yl)carbamoyl]-5-nitrobenzoic acid | 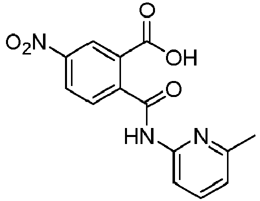 |
| 10 | 4-[(6-methylpyridin-2-yl)carbamoyl]-[1,1'-biphenyl]-3-carboxylic acid | 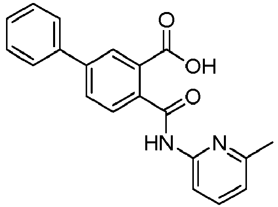 |
| 11 | 4-chloro-2-[(6-methylpyridin-2-yl)carbamoyl]benzoic acid | 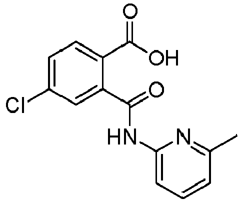 |
| 12 | 4-methyl-2-[(6-methylpyridin-2-yl)carbamoyl]benzoic acid | 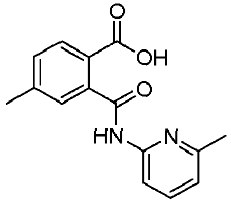 |
| 13 | 2-[(6-methylpyridin-2-yl)carbamoyl]-4-(trifluoromethyl)benzoic acid | 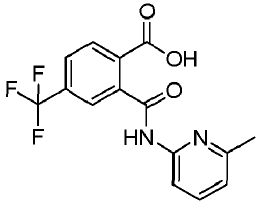 |
| 14 | 4,5-dichloro-2-[(6-methylpyridin-2-yl)carbamoyl]benzoic acid | 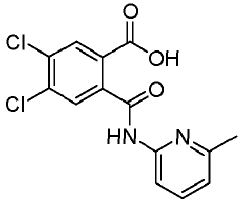 |
| 15 | 4,5-dimethyl-2-[(6-methylpyridin-2-yl)carbamoyl]benzoic acid | 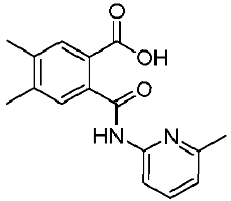 |
| 16 | 3-methyl-2-[(6-methylpyridin-2-yl)carbamoyl]benzoic acid | 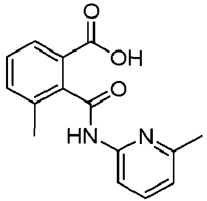 |
| 17 | 5-bromo-2-[(butan-2-yl)carbamoyl]benzoic acid | 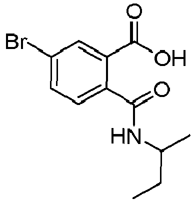 |
| 18 | 5-bromo-2-[(propan-2-yl)carbamoyl]benzoic acid | 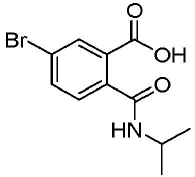 |
| 19 | 5-bromo-2-(phenylcarbamoyl)benzoic acid | 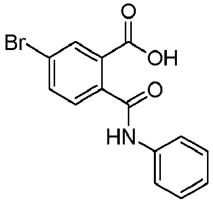 |
| 20 | 5-bromo-2-[(3-methylphenyl)carbamoyl]benzoic acid | 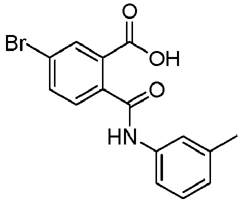 |
| 21 | 5-bromo-2-[(pyridin-2-yl)carbamoyl]benzoic acid | 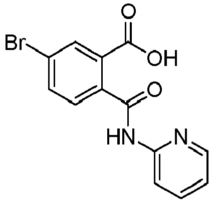 |
| 22 | 5-bromo-2-[(6-chloropyridin-2-yl)carbamoyl]benzoic acid | 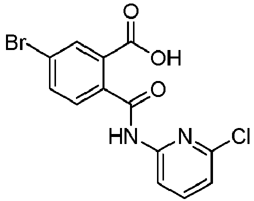 |
| 23 | 5-bromo-2-[(4-methylpyridin-2-yl)carbamoyl]benzoic acid | 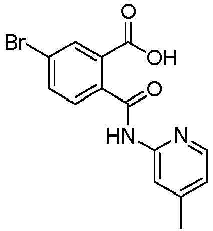 |
| 24 | 5-bromo-2-[(3-methylpyridin-2-yl)carbamoyl]benzoic acid | 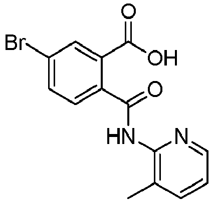 |
| 25 | 5-bromo-2-[(6-methylpyridin-3-yl)carbamoyl]benzoic acid | 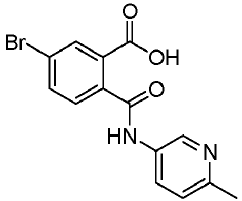 |
| 26 | 5-bromo-2-[(2-methylpyridin-4-yl)carbamoyl]benzoic acid | 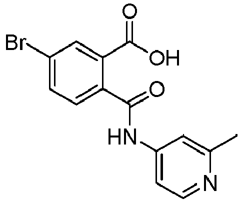 |
| 27 | 5-bromo-2-[(2-methylpyrimidin-4-yl)carbamoyl]benzoic acid | 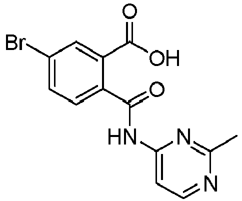 |
| 28 | 2-[(6-methoxypyridin-2-yl)carbamoyl]-5-(trifluoromethyl)benzoic acid | 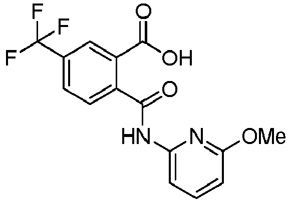 |
| 29 | 2-[(5,6-dimethylpyridin-2-yl)carbamoyl]-5-(trifluoromethyl)benzoic acid | 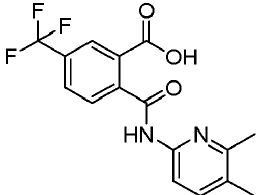 |
| 30 | 2-[(5,6,7,8-tetrahydroquinolin-2-yl)carbamoyl]-5-(trifluoromethyl)benzoic acid | 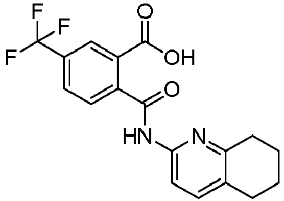 |

### BIOLOGICAL DATA

### Neurotensin scintillation proximity assay

The exemplified compounds of the invention were tested in a Neurotensin (NTS) scintillation proximity assay (SPA) and the IC₅₀ data is shown in the table below. NTS, which is a 13 amino acid neuropeptide, is a sortilin ligand. The IC₅₀ is a measure of the amount of the compound required to inhibit the binding of NTS to sortilin by 50%. The skilled person will recognise that the lower the IC₅₀ value, the less of the compound needed to achieve the desired effect, and as a result, the chances of undesirable off-target effects are reduced.

Compound affinity was determined by measuring the displacement of 3*H*-neurotensin binding to hSortilin in SPA format. Total volume of 40 µl in 50 mM HEPES pH 7.4 assay buffer containing 100 mM NaCl, 2.0 mM CaCI2, 0.1% BSA and 0.1% Tween-20. Compound pre-incubation for 30 min at RT with 150 nM of 6his-Sortilin before 5 nM [3*H*]-Neurotensin and Ni chelate imaging beads (Perkin Elmer) were added, after 6 h plate was read on a ViewLux with 360 s exposure time. Dose-response evaluation of compounds was performed with 10 concentrations of drugs (covering 3 decades). IC₅₀ values were calculated by nonlinear regression using the sigmoid concentration-response (variable slope) using Xlfit 4 (IDBS, UK). All values reported are average of at least 4 determinations.⁶

The data in the table below shows that the compounds disclosed herein are sortilin inhibitors.

| **Example** | **NTS IC₅₀(nM)** | **Example** | **NTS IC₅₀(nM)** |
|---|---|---|---|
| 1 | 710 | 16 | 43%* |
| 2 | 12000 | 17 | 30%* |
| 3 | 15%* | 18 | 35%* |
| 4 | 5%* | 19 | 69%* |
| 5 | 1400 | 20 | 5200 |
| 6 | 6400 | 21 | 3600 |
| 7 | 1100 | 22 | 1600 |
| 8 | 330 | 23 | 2400 |
| 9 | 1700 | 24 | 64%* |
| 10 | 2%* | 25 | 70%* |
| 11 | 69%* | 26 | 1800 |
| 12 | 57%* | 27 | 490 |
| 13 | 9100 | 28 | 1300 |
| 14 | 830 | 29 | 170 |
| 15 | 3400 | 30 | 100 |

| | | | |
|---|---|---|---|
| * % inhibition at 50 µM. | | | |

### Diabetic mouse model

Flash electroretinography (fERG) was used to measure the electrical response of the light sensitive cells of the retina in mice both prior to streptozocin (STZ) induction of diabetes (baseline) and 8 weeks after induction. Mice were exposed to increasing intensities of light flashes and the resulting electrical responses, measured in amplitude, were recorded. The skilled person will recognise that a cell with a higher measured amplitude is indicative of a healthy cell whilst cells with lower amplitudes is indicative of a damaged cell which is no longer functioning optimally. In the experimental group, the mice were administered with a polyclonal anti-sortilin antibody (2ul of a 1µg/µl solution in Dulbecco's Phosphate buffered saline) by intravitrial administration whereas in the control group the mice were administered with the vehicle only (Dulbecco's Phosphate buffered saline). All other variables, such as food intake, diet, exercise levels and exposure to environmental enrichment were kept the same between the two groups.

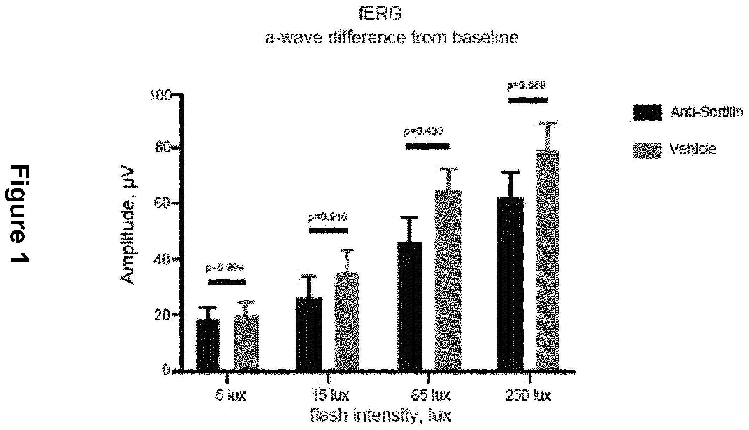

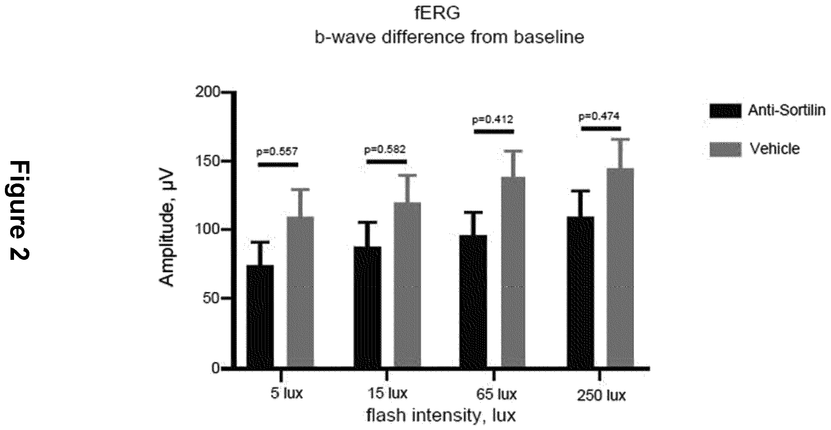

Figures 1 and 2 show the reduction in the fERG response 8 weeks after induction compared to the baseline for both the experimental group and the control group. As can be seen from the data, the reduction in fERG response was smaller for the experimental group compared to the control group. This shows that administration of the polyclonal anti-sortilin antibody was able to better protect the retinal cells of the mice from damage compared to the vehicle control group, and therefore suggestive of reducing disease progression. Without wishing to be bound by theory, it is thought that the polyclonal antibody competes with sortilin to bind to p75NTR thereby preventing activation of said receptor by sortilin.

Figure 1 shows the "a wave" component of the fERG response. Figure 2 shows the "b wave" component of the fERG response. This data demonstrates that interference with the p75NTR/sortilin/pro-neurotrophin and/or sortilin/pro-neurotrophin complex is effective across a range of photosensitive cell-types. The fact that the effect is not limited to a single cell-type is advantageous in that multiple cell-types can be targeted simultaneously and produce a more efficacious therapy to address diabetic retinopathy as a result.

The results of this study demonstrate that sortilin antagonists are useful in the prevention or treatment of diabetic retinopathy.

The study was repeated but modified such that the mice in the control group were administered twice with Example 8 of the application (a sortilin inhibitor) by intravitreal administration (2µl of a 1µg/µl solution in Dulbecco's Phosphate buffered saline), rather than with the polyclonal anti-sortilin antibody.

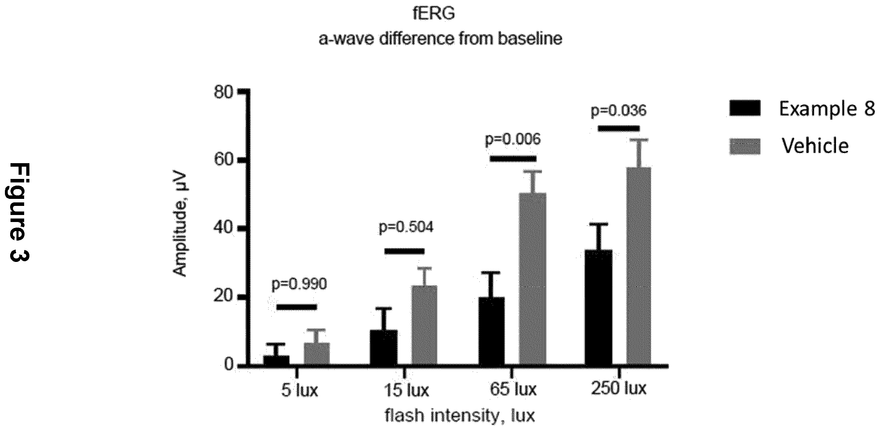

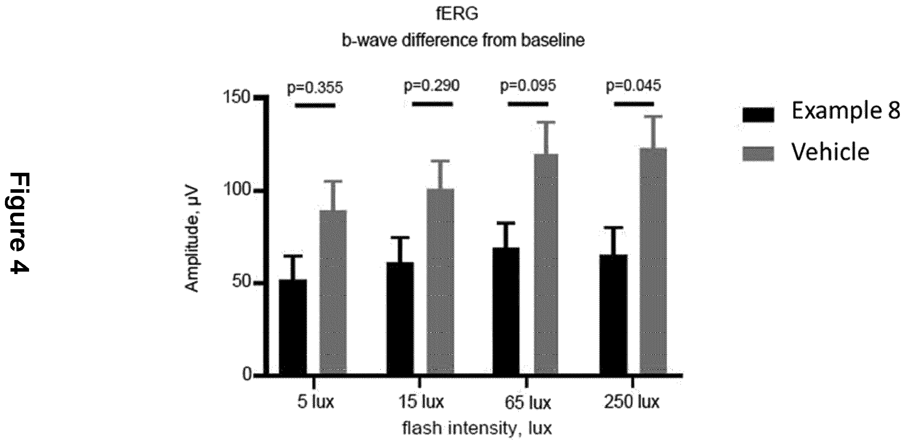

As can be seen from the data in Figures 3 and 4, the reduction in the fERG response 8 weeks after induction compared to the baseline was smaller for the experimental group compared to the control group. This shows that administration of the sortilin inhibitor was able to better protect the retinal cells of the mice from damage compared to the vehicle control group, and therefore suggestive of reducing disease progression. Surprisingly, the protective effect of the small molecule sortilin inhibitor (Example 8) was greater than that of the polyclonal antibody demonstrated in the first study.

The results of this study demonstrate that the compounds of Formula (I) and (II) disclosed herein are useful in the prevention or treatment of diabetic retinopathy.

### References

1. Wong, T.Y., et al., Diabetic retinopathy. Nat Rev Dis Primers, 2016. 2: p. 16012. NCD Risk Factor Collaboration (NCD-RisC). Worldwide trends in diabetes since 1980: a pooled analysis of 751 population-based studies with 4.4 million participants (vol 387, pg 1513, 2016). Lancet, 2017. 389(10068): p. E2-E2.
2. Antonetti, D.A., R. Klein, and T.W. Gardner, Diabetic retinopathy. N Engl J Med, 2012. 366(13): p. 1227-39.
3. Jansen, P., et al., Roles for the pro-neurotrophin receptor sortilin in neuronal development, aging and brain injury. Nature Neuroscience (2007), 10(11), pp.1449-1457.
4. Tenk, H.K., et al., ProBDNF induces neuronal apoptosis via activation of a receptor complex of p75NTR and sortilin. J Neuroscience (2005), 10(11), pp.1449-1457.
5. Nykjaer, A., et al., Sortilin is essential for proNGF-induced neuronal cell death. Nature (2004), 427(6977), pp.843-848.
6. T. J. Schrøder et al. The identification of AF38469: An orally bioavailable inhibitor of the VPS10P family sorting receptor Sortilin, Bioorganic & Medicinal Chemistry Letters 24 (2014) 177-180.

### Sequences referenced throughout the specification and forming part of the description.

SEQ ID NO: 1 (full length sortilin- isoform 1)

1 
51 
101 
151 
201 
251 
301 
351 
401 
451 
501 
551 
601 
651 
701 
751 
801 

SEQ ID NO: 2 (full length sortilin- isoform 2)

1 MERPWGAADG LSRWPHGLGL LLLLQLLPPS TLSQDRLDAP PPPAAPLPRW
51 SGPIGVSWGL RAAAAGGAFP RGGRWRRSAP GEDEECGRVR DFVAKLANNT
101 HQHVFDDLRG SVSLSWVGDS TGVILVLTTF HVPLVIMTFG QSKLYRSEDY
151 GKNFKDITDL INNTFIRTEF GMAIGPENSG KVVLTAEVSG GSRGGRIFRS
201 SDFAKNFVQT DLPFHPLTQM MYSPQNSDYL LALSTENGLW VSKNFGGKWE
251 EIHKAVCLAK WGSDNTIFFT TYANGSCTDL GALELWRTSD LGKSFKTIGV
301 KIYSFGLGGR FLFASVMADK DTTRRIHVST DQGDTWSMAQ LPSVGQEQFY
351 SILAANDDMV FMHVDEPGDT GFGTIFTSDD RGIVYSKSLD RHLYTTTGGE
401 TDFTNVTSLR GVYITSVLSE DNSIQTMITF DQGGRWTHLR KPENSECDAT
451 AKNKNECSLH IHASYSISQK LNVPMAPLSE PNAVGIVIAH GSVGDAISVM
501 VPDVYISDDG GYSWTKMLEG PHYYTILDSG GIIVAIEHSS RPINVIKFST
551 DEGQCWQTYT FTRDPIYFTG LASEPGARSM NISIWGFTES FLTSQWVSYT
601 IDFKDILERN CEEKDYTIWL AHSTDPEDYE DGCILGYKEQ FLRLRKSSVC
651 QNGRDYVVTK QPSICLCSLE DFLCDFGYYR PENDSKCVEQ PELKGHDLEF
701 CLYGREEHLT TNGYRKIPGD KCQGGVNPVR EVKDLKKKCT SNFLSPEKQN
751 SKSNSVPIIL AIVGLMLVTV VAGVLIVKKY VCGGRFLVHR YSVLQQHAEA
801 NGVDGVDALD TASHTNKSGY HDDSDEDLLE

SEQ ID NO: 3 (mature sortilin)

1 MTFGQSKLYR SEDYGKNFKD ITDLINNTFI RTEFGMAIGP ENSGKVVLTA
51 EVSGGSRGGR IFRSSDFAKN FVQTDLPFHP LTQMMYSPQN SDYLLALSTE
101 NGLWVSKNFG GKWEEIHKAV CLAKWGSDNT IFFTTYANGS CTDLGALELW
151 RTSDLGKSFK TIGVKIYSFG LGGRFLFASV MADKDTTRRI HVSTDQGDTW
201 SMAQLPSVGQ EQFYSILAAN DDMVFMHVDE PGDTGFGTIF TSDDRGIVYS
251 KSLDRHLYTT TGGETDFTNV TSLRGVYITS VLSEDNSIQT MITFDQGGRW
301 THLRKPENSE CDATAKNKNE CSLHIHASYS ISQKLNVPMA PLSEPNAVGI

361 VIAHGSVGDA ISVMVPDVYI SDDGGYSWTK MLEGPHYYTI LDSGGIIVAI
401 EHSSRPINVI KFSTDEGQCW QTYTFTRDPI YFTGLASEPG ARSMNISIWG
451 FTESFLTSQW VSYTIDFKDI LERNCEEKDY TIWLAHSTDP EDYEDGCILG
501 YKEQFLRLRK SSVCQNGRDY VVTKQPSICL CSLEDFLCDF GYYRPENDSK
551 CVEQPELKGH DLEFCLYGRE EHLTTNGYRK IPGDKCQGGV NPVREVKDLK
601 KKCTSNFLSP EKQNSKSNSV PIILAIVGLM LVTVVAGVLI VKKYVCGGRF
651 LVHRYSVLQQ HAEANGVDGV DALDTASHTN KSGYHDDSDE DLLE

## Claims

1. A sortilin antagonist for use in the prevention or treatment of diabetic retinopathy.

2. The sortilin antagonist for use according to claim 1, wherein
(i) the sortilin antagonist disrupts an interaction between a sortilin or sortilin-related molecule and a pro-neurotrophin molecule; and/or
(ii) the sortilin antagonist disrupts an interaction between a sortilin or sortilin-related molecule and a p75NTR molecule.

3. The sortilin antagonist for use according to claim 2, wherein the sortilin is mature sortilin or wherein the sortilin-related molecule is SorCS2.

4. The sortilin antagonist for use according to claim 1 to 3, wherein the sortilin antagonist is a sortilin inhibitor.

5. The sortilin antagonist for use according to any one of claims claim 1 to 4, wherein the sortilin antagonist has a molecular weight of <2000 Da, preferably wherein the sortilin antagonist has a molecular weight of <1000 Da.

6. The sortilin antagonist for use according to any one of claims 1 to 5, wherein the sortilin antagonist is a compound of Formula (I): 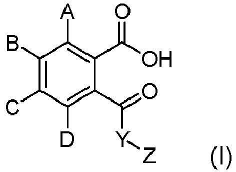 or a pharmaceutically acceptable salt, solvate, hydrate, geometrical isomer, tautomer, optical isomer, N-oxide, and/or prodrug thereof, wherein
Y is selected from the group consisting of -O-, -NR⁵-, and -S-;
Z is selected from the group consisting of optionally substituted C₁-C₆ alkyl, optionally substituted C₆-C₁₀ aryl, and optionally substituted C₁-C₉ heteroaryl;
A, B, C and D are each independently selected from the group consisting of H, optionally substituted C₁-C₆ alkyl, halo, NO₂, optionally substituted C₆-C₁₀ aryl, optionally substituted C₁-C₉ heteroaryl, -OR⁶, NR⁷R⁸, -SR⁹, -C(O)OR¹⁰,-C(O)NR¹¹R¹², -C(O)SR¹³, C(O₂)SR¹⁴;
R¹, R⁴, R⁵, R⁶, R⁹, R¹⁰, R¹³, and R¹⁴ are each independently selected from the group consisting of H and C₁-C₆ alkyl group, wherein the C₁-C₆ alkyl is optionally substituted with one or more halo atoms; and
R², R³, R⁷, R⁸, R¹¹, and R¹² are each independently selected from the group consisting of H and C₁-C₆ alkyl, wherein the C₁-C₆ alkyl is optionally substituted with one or more halo atoms, or R² and R³, R⁷ and R⁸, and/or R¹¹ and R¹² can be taken together with the nitrogen atom to which they are attached to from a 5- or 6- membered heterocycle, optionally substituted with one or more halo atoms;

7. The sortilin antagonist for use according to claim 6, wherein Y is -NR⁵-, preferably Y is -NH-.

8. The sortilin antagonist for use according to claim 6 or 7, wherein A and D are H.

9. The sortilin antagonist for use according to any one of claims claim 6 to 8, wherein B and C are independently selected from the group consisting of H, halo, CF₃, NO₂, and C₁-C₃ alkyl.

10. The sortilin antagonist for use according to any one of claims 6 to 9, wherein C is H.

11. The sortilin antagonist for use according to any one of claims 6 to 10, wherein B is selected from the group consisting of halo and CF₃, preferably wherein B is Br or CF₃.

12. The sortilin antagonist for use according to any one of claims 6 to 11, wherein Z is an optionally substituted C₆-C₁₀ aryl or an optionally substituted C₁-C₉ heteroaryl, more preferably Z is an optionally substituted phenyl, an optionally substituted pyridyl, or an optionally substituted pyrimidinyl.

13. The sortilin antagonist for use according to claim 12, wherein Z is substituted with one or more substituents independently selected from the group consisting of -OR¹⁵, halo, and C₁-C₄ alkyl, wherein R¹⁵ is H, halo, and C₁-C₃ alkyl, preferably wherein Z is substituted with one or more substituents selected from the group consisting of Cl, -OMe, and Me;
and/or wherein an alkylene group is attached to two adjacent atoms of group Z to form a 5- or 6-membered partially saturated or saturated ring, optionally wherein the alkylene group is substituted with one or more halo atoms.

14. The sortilin antagonist for use according to any preceding claim, wherein the sortilin antagonist is
• 2-[(6-methylpyridin-2-yl)carbamoyl]benzoic acid;
• 2-methyl-6-[(6-methylpyridin-2-yl)carbamoyl]benzoic acid;
• 5-bromo-2-[(6-methylpyridin-2-yl)carbamoyl]benzoic acid;
• 5-chloro-2-[(6-methylpyridin-2-yl)carbamoyl]benzoic acid;
• 5-methyl-2-[(6-methylpyridin-2-yl)carbamoyl]benzoic acid;
• 2-[(6-methylpyridin-2-yl)carbamoyl]-5-(propan-2-yl)benzoic acid;
• 2-[(6-methylpyridin-2-yl)carbamoyl]-5-(trifluoromethyl)benzoic acid;
• 2-[(6-methylpyridin-2-yl)carbamoyl]-5-nitrobenzoic acid;
• 4-[(6-methylpyridin-2-yl)carbamoyl]-[1,1'-biphenyl]-3-carboxylic acid;
• 4-bromo-2-[(6-methylpyridin-2-yl)carbamoyl]benzoic acid;
• 4-chloro-2-[(6-methylpyridin-2-yl)carbamoyl]benzoic acid;
• 4-methyl-2-[(6-methylpyridin-2-yl)carbamoyl]benzoic acid;
• 2-[(6-methylpyridin-2-yl)carbamoyl]-4-(trifluoromethyl)benzoic acid;
• 4,5-dichloro-2-[(6-methylpyridin-2-yl)carbamoyl]benzoic acid;
• 4,5-dimethyl-2-[(6-methylpyridin-2-yl)carbamoyl]benzoic acid;
• 3-methyl-2-[(6-methylpyridin-2-yl)carbamoyl]benzoic acid;
• 5-bromo-2-[(butan-2-yl)carbamoyl]benzoic acid;
• 5-bromo-2-[(propan-2-yl)carbamoyl]benzoic acid;
• 5-bromo-2-(phenylcarbamoyl)benzoic acid;
• 5-bromo-2-[(3-methylphenyl)carbamoyl]benzoic acid;
• 5-bromo-2-[(pyridin-2-yl)carbamoyl]benzoic acid;
• 5-bromo-2-[(6-chloropyridin-2-yl)carbamoyl]benzoic acid;
• 5-bromo-2-[(4-methylpyridin-2-yl)carbamoyl]benzoic acid;
• 5-bromo-2-[(3-methylpyridin-2-yl)carbamoyl]benzoic acid;
• 5-bromo-2-[(6-methylpyridin-3-yl)carbamoyl]benzoic acid;
• 5-bromo-2-[(2-methylpyridin-4-yl)carbamoyl]benzoic acid;
• 5-bromo-2-[(2-methylpyrimidin-4-yl)carbamoyl]benzoic acid;
• 2-[(6-methoxypyridin-2-yl)carbamoyl]-5-(trifluoromethyl)benzoic acid;
• 2-[(5,6-dimethylpyridin-2-yl)carbamoyl]-5-(trifluoromethyl)benzoic acid;
• 2-[(5,6,7,8-tetrahydroquinolin-2-yl)carbamoyl]-5-(trifluoromethyl)benzoic acid,
or a pharmaceutically acceptable salt, solvate, hydrate, tautomer, optical isomer, N-oxide, and/or prodrug thereof, preferably the sortilin antagonist is
• 5-bromo-2-[(6-methylpyridin-2-yl)carbamoyl]benzoic acid;
• 5-chloro-2-[(6-methylpyridin-2-yl)carbamoyl]benzoic acid;
• 5-methyl-2-[(6-methylpyridin-2-yl)carbamoyl]benzoic acid;
• 2-[(6-methylpyridin-2-yl)carbamoyl]-5-(propan-2-yl)benzoic acid;
• 2-[(6-methylpyridin-2-yl)carbamoyl]-5-(trifluoromethyl)benzoic acid;
• 2-[(6-methylpyridin-2-yl)carbamoyl]-5-nitrobenzoic acid;
• 4-bromo-2-[(6-methylpyridin-2-yl)carbamoyl]benzoic acid;
• 2-[(6-methylpyridin-2-yl)carbamoyl]-4-(trifluoromethyl)benzoic acid;
• 4,5-dichloro-2-[(6-methylpyridin-2-yl)carbamoyl]benzoic acid;
• 4,5-dimethyl-2-[(6-methylpyridin-2-yl)carbamoyl]benzoic acid;
• 5-bromo-2-[(3-methylphenyl)carbamoyl]benzoic acid;
• 5-bromo-2-[(pyridin-2-yl)carbamoyl]benzoic acid;
• 5-bromo-2-[(6-chloropyridin-2-yl)carbamoyl]benzoic acid;
• 5-bromo-2-[(4-methylpyridin-2-yl)carbamoyl]benzoic acid;
• 5-bromo-2-[(2-methylpyridin-4-yl)carbamoyl]benzoic acid;
• 5-bromo-2-[(2-methylpyrimidin-4-yl)carbamoyl]benzoic acid;
• 2-[(6-methoxypyridin-2-yl)carbamoyl]-5-(trifluoromethyl)benzoic acid;
• 2-[(5,6-dimethylpyridin-2-yl)carbamoyl]-5-(trifluoromethyl)benzoic acid;
• 2-[(5,6,7,8-tetrahydroquinolin-2-yl)carbamoyl]-5-(trifluoromethyl)benzoic acid;
or a pharmaceutically acceptable salt, solvate, hydrate, tautomer, optical isomer, N-oxide, and/or prodrug thereof.

15. A pharmaceutical formulation comprising a sortilin antagonist as defined in any of claims 1 to 14 and one or more pharmaceutically acceptable carriers or excipients, for use in the treatment or prevention of diabetic retinopathy, preferably wherein the formulation is an ophthalmic formulation.


SEQUENCE LISTING

<110>  Insusense ApS

<120>  COMPOUNDS

<130>  RMC00172EP

<160>  3

<170>  PatentIn version 3.5

<210>  1
<211>  831
<212>  PRT
<213>  Homo sapiens

<400>  1

<210>  2
<211>  830
<212>  PRT
<213>  Homo sapiens

<400>  2

Met Glu Arg Pro Trp Gly Ala Ala Asp Gly Leu Ser Arg Trp Pro His
1               5                   10                  15

Gly Leu Gly Leu Leu Leu Leu Leu Gln Leu Leu Pro Pro Ser Thr Leu
            20                  25                  30

Ser Gln Asp Arg Leu Asp Ala Pro Pro Pro Pro Ala Ala Pro Leu Pro
        35                  40                  45

Arg Trp Ser Gly Pro Ile Gly Val Ser Trp Gly Leu Arg Ala Ala Ala
    50                  55                  60

Ala Gly Gly Ala Phe Pro Arg Gly Gly Arg Trp Arg Arg Ser Ala Pro
65                  70                  75                  80

Gly Glu Asp Glu Glu Cys Gly Arg Val Arg Asp Phe Val Ala Lys Leu
                85                  90                  95

Ala Asn Asn Thr His Gln His Val Phe Asp Asp Leu Arg Gly Ser Val
            100                 105                 110

Ser Leu Ser Trp Val Gly Asp Ser Thr Gly Val Ile Leu Val Leu Thr
        115                 120                 125

Thr Phe His Val Pro Leu Val Ile Met Thr Phe Gly Gln Ser Lys Leu
    130                 135                 140

Tyr Arg Ser Glu Asp Tyr Gly Lys Asn Phe Lys Asp Ile Thr Asp Leu
145                 150                 155                 160

Ile Asn Asn Thr Phe Ile Arg Thr Glu Phe Gly Met Ala Ile Gly Pro
                165                 170                 175

Glu Asn Ser Gly Lys Val Val Leu Thr Ala Glu Val Ser Gly Gly Ser
            180                 185                 190

Arg Gly Gly Arg Ile Phe Arg Ser Ser Asp Phe Ala Lys Asn Phe Val
        195                 200                 205

Gln Thr Asp Leu Pro Phe His Pro Leu Thr Gln Met Met Tyr Ser Pro
    210                 215                 220

Gln Asn Ser Asp Tyr Leu Leu Ala Leu Ser Thr Glu Asn Gly Leu Trp
225                 230                 235                 240

Val Ser Lys Asn Phe Gly Gly Lys Trp Glu Glu Ile His Lys Ala Val
                245                 250                 255

Cys Leu Ala Lys Trp Gly Ser Asp Asn Thr Ile Phe Phe Thr Thr Tyr
            260                 265                 270

Ala Asn Gly Ser Cys Thr Asp Leu Gly Ala Leu Glu Leu Trp Arg Thr
        275                 280                 285

Ser Asp Leu Gly Lys Ser Phe Lys Thr Ile Gly Val Lys Ile Tyr Ser
    290                 295                 300

Phe Gly Leu Gly Gly Arg Phe Leu Phe Ala Ser Val Met Ala Asp Lys
305                 310                 315                 320

Asp Thr Thr Arg Arg Ile His Val Ser Thr Asp Gln Gly Asp Thr Trp

                325                 330                 335

Ser Met Ala Gln Leu Pro Ser Val Gly Gln Glu Gln Phe Tyr Ser Ile
            340                 345                 350

Leu Ala Ala Asn Asp Asp Met Val Phe Met His Val Asp Glu Pro Gly
        355                 360                 365

Asp Thr Gly Phe Gly Thr Ile Phe Thr Ser Asp Asp Arg Gly Ile Val
    370                 375                 380

Tyr Ser Lys Ser Leu Asp Arg His Leu Tyr Thr Thr Thr Gly Gly Glu
385                 390                 395                 400

Thr Asp Phe Thr Asn Val Thr Ser Leu Arg Gly Val Tyr Ile Thr Ser
                405                 410                 415

Val Leu Ser Glu Asp Asn Ser Ile Gln Thr Met Ile Thr Phe Asp Gln
            420                 425                 430

Gly Gly Arg Trp Thr His Leu Arg Lys Pro Glu Asn Ser Glu Cys Asp
        435                 440                 445

Ala Thr Ala Lys Asn Lys Asn Glu Cys Ser Leu His Ile His Ala Ser
    450                 455                 460

Tyr Ser Ile Ser Gln Lys Leu Asn Val Pro Met Ala Pro Leu Ser Glu
465                 470                 475                 480

Pro Asn Ala Val Gly Ile Val Ile Ala His Gly Ser Val Gly Asp Ala
                485                 490                 495

Ile Ser Val Met Val Pro Asp Val Tyr Ile Ser Asp Asp Gly Gly Tyr
            500                 505                 510

Ser Trp Thr Lys Met Leu Glu Gly Pro His Tyr Tyr Thr Ile Leu Asp
        515                 520                 525

Ser Gly Gly Ile Ile Val Ala Ile Glu His Ser Ser Arg Pro Ile Asn
    530                 535                 540

Val Ile Lys Phe Ser Thr Asp Glu Gly Gln Cys Trp Gln Thr Tyr Thr
545                 550                 555                 560

Phe Thr Arg Asp Pro Ile Tyr Phe Thr Gly Leu Ala Ser Glu Pro Gly
                565                 570                 575

Ala Arg Ser Met Asn Ile Ser Ile Trp Gly Phe Thr Glu Ser Phe Leu
            580                 585                 590

Thr Ser Gln Trp Val Ser Tyr Thr Ile Asp Phe Lys Asp Ile Leu Glu
        595                 600                 605

Arg Asn Cys Glu Glu Lys Asp Tyr Thr Ile Trp Leu Ala His Ser Thr
    610                 615                 620

Asp Pro Glu Asp Tyr Glu Asp Gly Cys Ile Leu Gly Tyr Lys Glu Gln
625                 630                 635                 640

Phe Leu Arg Leu Arg Lys Ser Ser Val Cys Gln Asn Gly Arg Asp Tyr
                645                 650                 655

Val Val Thr Lys Gln Pro Ser Ile Cys Leu Cys Ser Leu Glu Asp Phe
            660                 665                 670

Leu Cys Asp Phe Gly Tyr Tyr Arg Pro Glu Asn Asp Ser Lys Cys Val
        675                 680                 685

Glu Gln Pro Glu Leu Lys Gly His Asp Leu Glu Phe Cys Leu Tyr Gly
    690                 695                 700

Arg Glu Glu His Leu Thr Thr Asn Gly Tyr Arg Lys Ile Pro Gly Asp
705                 710                 715                 720

Lys Cys Gln Gly Gly Val Asn Pro Val Arg Glu Val Lys Asp Leu Lys
                725                 730                 735

Lys Lys Cys Thr Ser Asn Phe Leu Ser Pro Glu Lys Gln Asn Ser Lys
            740                 745                 750

Ser Asn Ser Val Pro Ile Ile Leu Ala Ile Val Gly Leu Met Leu Val
        755                 760                 765

Thr Val Val Ala Gly Val Leu Ile Val Lys Lys Tyr Val Cys Gly Gly
    770                 775                 780

Arg Phe Leu Val His Arg Tyr Ser Val Leu Gln Gln His Ala Glu Ala
785                 790                 795                 800

Asn Gly Val Asp Gly Val Asp Ala Leu Asp Thr Ala Ser His Thr Asn
                805                 810                 815

Lys Ser Gly Tyr His Asp Asp Ser Asp Glu Asp Leu Leu Glu
            820                 825                 830

<210>  3
<211>  694
<212>  PRT
<213>  Homo sapiens

<400>  3

Met Thr Phe Gly Gln Ser Lys Leu Tyr Arg Ser Glu Asp Tyr Gly Lys
1               5                   10                  15

Asn Phe Lys Asp Ile Thr Asp Leu Ile Asn Asn Thr Phe Ile Arg Thr
            20                  25                  30

Glu Phe Gly Met Ala Ile Gly Pro Glu Asn Ser Gly Lys Val Val Leu
        35                  40                  45

Thr Ala Glu Val Ser Gly Gly Ser Arg Gly Gly Arg Ile Phe Arg Ser
    50                  55                  60

Ser Asp Phe Ala Lys Asn Phe Val Gln Thr Asp Leu Pro Phe His Pro
65                  70                  75                  80

Leu Thr Gln Met Met Tyr Ser Pro Gln Asn Ser Asp Tyr Leu Leu Ala
                85                  90                  95

Leu Ser Thr Glu Asn Gly Leu Trp Val Ser Lys Asn Phe Gly Gly Lys
            100                 105                 110

Trp Glu Glu Ile His Lys Ala Val Cys Leu Ala Lys Trp Gly Ser Asp
        115                 120                 125

Asn Thr Ile Phe Phe Thr Thr Tyr Ala Asn Gly Ser Cys Thr Asp Leu
    130                 135                 140

Gly Ala Leu Glu Leu Trp Arg Thr Ser Asp Leu Gly Lys Ser Phe Lys
145                 150                 155                 160

Thr Ile Gly Val Lys Ile Tyr Ser Phe Gly Leu Gly Gly Arg Phe Leu
                165                 170                 175

Phe Ala Ser Val Met Ala Asp Lys Asp Thr Thr Arg Arg Ile His Val
            180                 185                 190

Ser Thr Asp Gln Gly Asp Thr Trp Ser Met Ala Gln Leu Pro Ser Val
        195                 200                 205

Gly Gln Glu Gln Phe Tyr Ser Ile Leu Ala Ala Asn Asp Asp Met Val
    210                 215                 220

Phe Met His Val Asp Glu Pro Gly Asp Thr Gly Phe Gly Thr Ile Phe
225                 230                 235                 240

Thr Ser Asp Asp Arg Gly Ile Val Tyr Ser Lys Ser Leu Asp Arg His
                245                 250                 255

Leu Tyr Thr Thr Thr Gly Gly Glu Thr Asp Phe Thr Asn Val Thr Ser
            260                 265                 270

Leu Arg Gly Val Tyr Ile Thr Ser Val Leu Ser Glu Asp Asn Ser Ile
        275                 280                 285

Gln Thr Met Ile Thr Phe Asp Gln Gly Gly Arg Trp Thr His Leu Arg
    290                 295                 300

Lys Pro Glu Asn Ser Glu Cys Asp Ala Thr Ala Lys Asn Lys Asn Glu
305                 310                 315                 320

Cys Ser Leu His Ile His Ala Ser Tyr Ser Ile Ser Gln Lys Leu Asn
                325                 330                 335

Val Pro Met Ala Pro Leu Ser Glu Pro Asn Ala Val Gly Ile Val Ile
            340                 345                 350

Ala His Gly Ser Val Gly Asp Ala Ile Ser Val Met Val Pro Asp Val
        355                 360                 365

Tyr Ile Ser Asp Asp Gly Gly Tyr Ser Trp Thr Lys Met Leu Glu Gly
    370                 375                 380

Pro His Tyr Tyr Thr Ile Leu Asp Ser Gly Gly Ile Ile Val Ala Ile
385                 390                 395                 400

Glu His Ser Ser Arg Pro Ile Asn Val Ile Lys Phe Ser Thr Asp Glu
                405                 410                 415

Gly Gln Cys Trp Gln Thr Tyr Thr Phe Thr Arg Asp Pro Ile Tyr Phe
            420                 425                 430

Thr Gly Leu Ala Ser Glu Pro Gly Ala Arg Ser Met Asn Ile Ser Ile
        435                 440                 445

Trp Gly Phe Thr Glu Ser Phe Leu Thr Ser Gln Trp Val Ser Tyr Thr
    450                 455                 460

Ile Asp Phe Lys Asp Ile Leu Glu Arg Asn Cys Glu Glu Lys Asp Tyr
465                 470                 475                 480

Thr Ile Trp Leu Ala His Ser Thr Asp Pro Glu Asp Tyr Glu Asp Gly
                485                 490                 495

Cys Ile Leu Gly Tyr Lys Glu Gln Phe Leu Arg Leu Arg Lys Ser Ser
            500                 505                 510

Val Cys Gln Asn Gly Arg Asp Tyr Val Val Thr Lys Gln Pro Ser Ile
        515                 520                 525

Cys Leu Cys Ser Leu Glu Asp Phe Leu Cys Asp Phe Gly Tyr Tyr Arg
    530                 535                 540

Pro Glu Asn Asp Ser Lys Cys Val Glu Gln Pro Glu Leu Lys Gly His
545                 550                 555                 560

Asp Leu Glu Phe Cys Leu Tyr Gly Arg Glu Glu His Leu Thr Thr Asn
                565                 570                 575

Gly Tyr Arg Lys Ile Pro Gly Asp Lys Cys Gln Gly Gly Val Asn Pro
            580                 585                 590

Val Arg Glu Val Lys Asp Leu Lys Lys Lys Cys Thr Ser Asn Phe Leu
        595                 600                 605

Ser Pro Glu Lys Gln Asn Ser Lys Ser Asn Ser Val Pro Ile Ile Leu
    610                 615                 620

Ala Ile Val Gly Leu Met Leu Val Thr Val Val Ala Gly Val Leu Ile
625                 630                 635                 640

Val Lys Lys Tyr Val Cys Gly Gly Arg Phe Leu Val His Arg Tyr Ser
                645                 650                 655

Val Leu Gln Gln His Ala Glu Ala Asn Gly Val Asp Gly Val Asp Ala
            660                 665                 670

Leu Asp Thr Ala Ser His Thr Asn Lys Ser Gly Tyr His Asp Asp Ser
        675                 680                 685

Asp Glu Asp Leu Leu Glu
    690